Europäisches Patentamt

(19) European Patent Office

Office européen des brevets

(11) Publication number: **0 105 845**

**A1**

(12) **EUROPEAN PATENT APPLICATION**

(21) Application number: **83830006.9**

(22) Date of filing: **19.01.83**

(51) Int. Cl.³: **A 61 M 1/03**

(30) Priority: **20.01.82 IT 4000482**

(43) Date of publication of application:
**18.04.84 Bulletin 84/16**

(84) Designated Contracting States:
**BE DE FR GB LU NL**

(71) Applicant: **HAEMOTRONIC srl.**
**Via Morandi 4**
**I-41037 Mirandola (Modena)(IT)**

(72) Inventor: **Malavasi, Giancarlo**
**Via Cavalieri di Vittorio Veneto 15**
**Mirandola (MO)(IT)**

(74) Representative: **Massari, Marcello**
**Studio M. Massari S.r.l. 23, Via Fontanella Borghese**
**I-00186 Roma(IT)**

(54) **Apparatus for pumping the blood in extracorporeal dialysis through an artificial kidney.**

(57) A pumping apparatus to be associated to a pneumatic machine (PM), for pumping the blood in extra-corporeal dialysis through an artificial kidney comprising a dialyzer (DL), a drawing chamber (DC) and a needle (NL) to be introduced into the vein of the patient, wherein no crushing of the blood corpucles takes place, thus avoiding any blood alteration, which apparatus comprises a rigid housing (10) defining an inner cavity and a resilient membrane (11) dividing the cavity into two separate chambers, (18, 19) one (18) connected to the dialyzer (DL) and the other (19) connected to the pneumatic machine (PM), the pressure or vacuum created in the other chamber (19) by the pneumatic machine, (PM) deflects alternatively the resilient membrane (11) in and out of this chamber (19) causing the volume of the one chamber (18) to vary, thus causing the one chamber (18) to pump the blood in and out of the patient to and from dialyzer (DL).

FIG.2

EP 0 105 845 A1

1

"APPARATUS FOR PUMPING THE BLOOD IN EXTRACORPOREAL DIALYSIS THROUGH AN ARTIFICIAL KIDNEY"

This invention relates to an apparatus for pumping the blood especially apted to be used in extracorporeal dialysis through an artificial kidney.

All known apparatus used for pumping the blood, for instance in extracorporeal dialysis, even if able to operate satisfactorily may often cause alterations of the blood owing to the crushing of the blood corpucles.

This crushing for instance is likely to take place in the pumps used at present for pumping the blood in the extracorporeal dialysis system, namely peristaltic pumps.

This invention is intended to provide a pumping apparatus wherein this drawback is overcome.

The pumping apparatus of the invention comprises a rigid housing consisting of two identical shells or caps of elongated form joined at their peripheral edges, which edges sealingly hold therebetween the periphery of a resilient membrane dividing the space inside the housing into two identical chambers.

Each elongated cap has two pipe fittings.

The apparatus of invention is shown in a preferred application wherein the same is connected in the circuit of a extracorporeal dialysis sistem between a pneumatic machine and dialyzer.

More precisely a first chamber is connected to the dialyzer through a pipe fitting thereof and the other chamber is connected to the pneumatic machine through a pipe fitting thereof. The other pipe fitting of the one chamber can be used to introduce any proper medicine into the blood while the other pipe fitting of the other chamber can be connected to any suitable instrument.

In operation, the pneumatic machine created alternatively a vacuum and a pressure in the other chamber causing the resilient membrane to deflect in and out in respect of this chamber.

Consequently the volume of the first chamber is correspondingly increased or descreased alternatively.

This variation of the volume of the first chamber causes the blood of the patient, to be subjected to extracorporeal dialysis and connected to the dialyzer through a drawing chamber, to be sucked into the dialyzer, to be dialyzed and then to be returned to the patient.

The apparatus of the invention has one more advantageous feature, namely that only a needle can be used both for drawing the blood out of the patient and returning the same to the latter after the dialysis has taken place.

The apparatus of the invention will be now described more particularly with reference to the accompayning drawing wherein:

fig. 1 is a sectional view of the apparatus; and

fig. 2 shows diagrammatically an extracorporeal dialysis system including the apparatus shown in fig. 1.

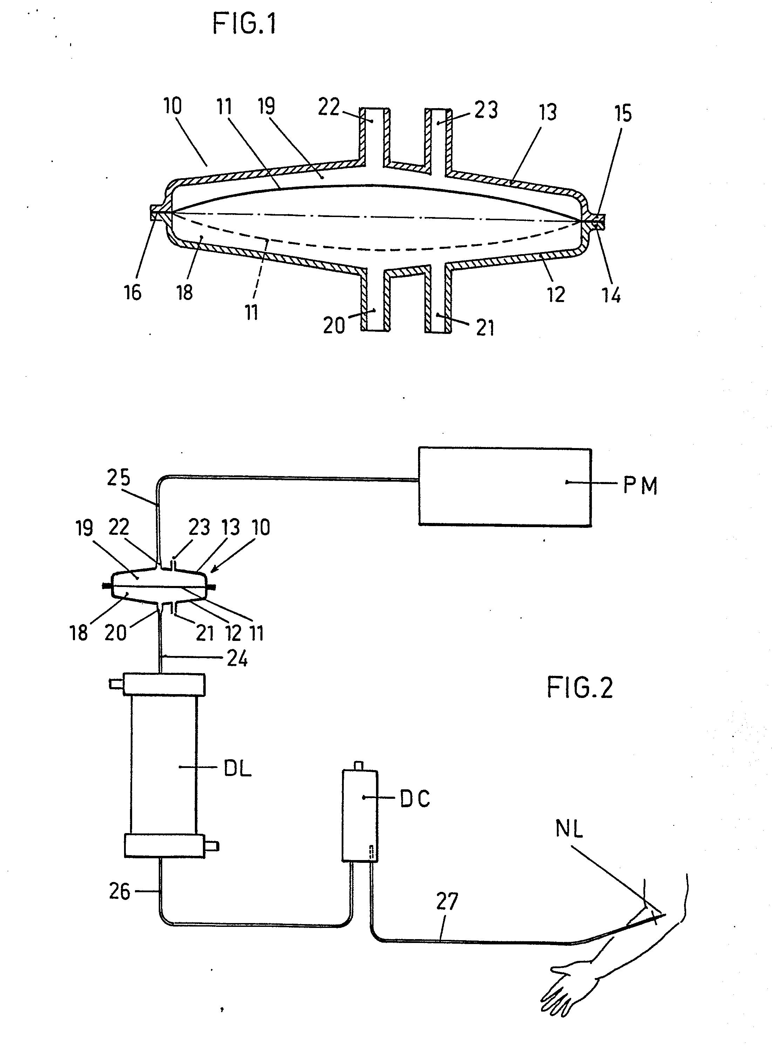

Referring firstly to fig. 1 the pumping apparatus of the invention comprises substantially a rigid housing 10 and an inner membrane 11.

Rigid housing 10 consists of a first rigid elongated cap 12 and a second rigid elongated cap 13 identical with cap 12.

The peripheral edge 14 of cap 12 and peripheral edge 15 of cap 13 are suitably firmly joined together and they sealingly hold therebetween the periphery 16 of resilient membrane 11, consequently resilient membrane 11 divides the inner space of the housing into two identical chambers referred to by numerals 18 and 19.

First cap 12 has two pipe fittings referred to by references 20 and 21 and second cap 13 has two pipe fittings referred to by references 22 and 23.

As illustrated in fig. 2, chamber 18 of the apparatus of the invention is connected to dialyzer DL for instance, through pipe fitting 20 and hose 24, and chamber 19 is connected to pneumatic machine PM, for instance, through pipe fitting 22 and hose 25.

As shown for sake of completeness, dialyzer DL is connected to a vein of the patient arm PA by means of a needle NL through drawing chamber DC.

As stated above, pipe fitting 21 can be used to introduce in the patient blood any proper medicine while pipe fitting 23 can be used for connecting chamber 19 to any suitable instrument, as a manometer.

In operation, pneumatic machine PM timely creates, alternatively, a vacuum and a pressure in chamber 19 through hose 25 and consequently resilient membrane 11 is alternatively deflected in this chamber, (as shown in full line) and out thereof, (as shown in dotted line).

These deflections of resilient membrane 11 alternatively increase and decrease the volume of chamber 18 that is sealed off chamber 19, which chamber 18 consequently performs a pumping action on the blood of the patient through hose 26, drawing chamber DC, hose 27 and needle NL.

The operation of pneumatic machine PM and dialyzer DL is timed and accordingly the blood to be dialyzed is sucked into dialyzer DL, dialyzed therein, and then returned to the patient.

It will be appreciated that by making use of the apparatus of the invention, the blood circulation, i.e. chamber 18, hose 24 dialyzer DL , hose 25, drawing chamber DC hose 27, needle NL, is completely separate from the gas circulation, i.e. chamber 19, hose 25 and pneumatic machine PM.

Furthermore it will be appreciated that the simmetric shape of caps 12 and 13 makes the apparatus capable to be inverted in the dialysis system, i.e. chamber 18 can be connected to pneumatic machine PM and chamber 19 can be connected to dialyzer DL without affecting the apparatus operation. This feature makes it impossible to make mistakes in connecting the apparatus to the dialyzer system.

5

The pneumatic machine PM, dialyzer DL and drawing chamber DC are well known in the art and
will not be described here.

It will be understood that the structure details
of this apparatus can be modified in respect of
what setforth and partially illustrated, without departing from the scope and concepts of the inven-
tion.

CLAIMS:

1. Apparatus for pumping the blood especially apted to be used in association to a pneumatic machine (PM) in extracorporeal dialysis through an artificial kidney, which kidney includes a dialyzer (DL) and a hollow needle (NL) to be introduced in a vein of a patient, said apparatus comprising a rigid housing (10) defining an inner cavity, a flexible membrane (11) impervious to fluid mounted in an intermediate position inside said housing (10), dividing said inner cavity into two chambers (18,19), first and second pipe fitting means (20, 21) on one side of said rigid housing (10) for connecting outside elements to one of said two chambers, and first and second (22, 23) pipe fitting means on the opposite side of said housing (10) for connecting outside elements to the other of said two chambers (18, 19).

2. The apparatus of claim 1, wherein said rigid housing (10) comprises two rigid elongated caps (12,13) identical with each other, sealingly joined at their peripheral edges (14, 15).

3. The apparatus of claim 2, wherein said flexible membrane (11) has the periphery (16) thereof sealingly held between said peripheral edges (14, 15) of said rigid elongated caps (12, 13) and accordingly said membrane (11) defines a first chamber (18) in one (12) of said caps and the second chamber (19) in the other cap (13).

4. The apparatus of claim 3, wherein each of said caps (12, 13) has formed thereon said first and second pipe fitting means (21, 22, 23, 24).

5. The apparatus of claim 4, wherein said first pipe fitting (21) of the one cap (12) is connected to said dialyzer (DL) ond said second pipe fitting (22) is apted to be connected to a suitable medical instrument for introducing any proper medicine into the patient blood.

6. The apparatus of claim 4, wherein said first pipe fitting (22) of the other cap (13) is connected to said pneumatic machine (PM) and said second pipe fitting (23) is apted to be connected to a suitable instrument as a manometer.

0105845

FIG.1

FIG.2

0105845

| European Patent Office | EUROPEAN SEARCH REPORT | Application number |
|---|---|---|
| | | EP 83 83 0006 |

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | US-A-4 071 444 (PURDUE) * Whole document, in particular claims 1,2; column 2, lines 44-46; column 4, lines 7-15 * | 1-3 | A 61 M 1/03 |
| A | | 5,6 | |
| Y | US-A-3 892 236 (DJERASSI) * Figure 1; column 2, lines 54-58; column 2, line 64 - column 3, line 16 * | 1-3 | |
| Y | US-A-4 135 496 (CHAZOV) * Column 1, lines 41-44; figure 1; column 3, lines 17-30, 52-53 * | 1-3 | |
| A | | 6 | TECHNICAL FIELDS SEARCHED (Int. Cl. 3) |
| A | US-A-3 606 592 (BENDIX) * Figure 4 * | 4 | A 61 M |
| A | FR-A-2 258 192 (SANDOZ) * Page 4, lines 21-25; page 5, lines 15-17, 31-32 * | 5,6 | |

The present search report has been drawn up for all claims

| Place of search | Date of completion of the search | Examiner |
|---|---|---|
| THE HAGUE | 06-01-1984 | PETZUCH M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82